# EUROPEAN PATENT APPLICATION

(11) **EP 4 726 032 A1**
(43) Date of publication of application: **15.04.2026**
(21) Application number: 24818812.0
(22) Date of filing: 07.06.2024
(51) Int. Cl.: C12N 7/00, C12N 15/869, A61K 35/763, A61K 48/00, A61K 38/20, A61K 38/19, A61K 39/395, A61P 35/00, C12R 1/93

(54) **ONCOLYTIC HSV-1 CLINICAL ISOLATE, DIRECTED EVOLUTION STRAIN, INFECTIOUS CLONE AND USE**

(30) Priority: 08.06.2023 CN 202310678456
(71) Applicant: Wuxi Biologics (Shanghai FX) Co., Ltd., Shanghai 201403 (CN)
(72) Inventor: HAO, Mengru, Shanghai 201403 (CN); ZHOU, Yang, Shanghai 201403 (CN); REN, Xuqian, Shanghai 201403 (CN); WU, Ke, Shanghai 201403 (CN); BU, Yan, Shanghai 201403 (CN); NI, Longquan, Shanghai 201403 (CN); ZHENG, Yong, Shanghai 201403 (CN)
(74) Representative: Modiano, Gabriella Diana
(86) International application number: PCT/CN2024/098281
(87) International publication number: WO 2024/251286

(57) **Abstract**

The present disclosure relates to an oncolytic HSV-1 clinical isolate, a directed evolution strain, an infectious clone and a use. Specifically, provided is an isolated HSV-1 clinical virus strain which has a significantly superior tumor cell killing capability against tumor cells than commercially available HSV-1 strains. Also provided are an evolved strain obtained by directed evolution of the clinical virus strain, an infectious clone containing viral genetic information, and a use of an active substance in tumor prevention and treatment and as an exogenous gene carrier.

## Description

### TECHNICAL FIELD

The present disclosure belongs to the fields of biotechnology and medicine, particularly in the field of tumor treatment. Specifically, the present disclosure relates to newly isolated and/or directed evolution-modified HSV clinical virus strain with excellent oncolytic properties, infectious clone, and use thereof.

### BACKGROUND

Since the advent of molecular biology, viruses have gradually transformed from pathogenic microorganisms into vectors with wide applications in the biomedical field. The ability of viruses to precisely enter specific tissues and cells and efficiently express exogenous genes within these cells is unmatched by other vectors such as nanoparticles, for example, lipid nanoparticles (LNPs), dendrimer, polymer, and exosomes. Based on these characteristics, viral vectors are increasingly used in gene therapy and cancer treatment. Among them, oncolytic viruses, which rely on the high specificity of viruses in killing cancer cells, have become a highly promising area of research in cancer treatment.

Over the decades, more than a dozen viruses from different families have been engineered into oncolytic viruses. Among them, herpes simplex virus (HSV), with its high exogenous gene load, broad-spectrum killing ability against cancer cells, and reliable genetic stability, has become one of the most popular oncolytic viruses with the most clinical applications. Currently, the only oncolytic virus approved by the FDA for the treatment of melanoma, T-Vec (talimogene laherparepve), is derived from HSV oncolytic virus, and another drug conditionally marketed in Japan for the treatment of glioblastoma, Delytact (teserpaturev/G47Δ), is also an HSV oncolytic virus. This demonstrates the value of HSV as an oncolytic virus.

Common modifications to HSV to selectively replicate and kill tumors include, but are not limited to, deleting non- essential genes such as ICP34.5, ICP6, and US47, with the deletion of the neurotoxic gene ICP34.5 being the most widespread. While deleting these non-essential genes reduces the toxicity of HSV to cancer cells, it reduces its ability to kill normal tissues and cells even more significantly. This allows HSV to kill cancerous tissues at doses that do not kill normal tissues and cells, thus creating a "therapeutic window." Furthermore, it can activate the immune system through the release of viral and cancer cell antigens, achieving sustained tumor killing.

However, though HSV oncolytic viruses are already on the market and in large numbers in clinical trials, most of them use laboratory strains that have been passaged in the lab for decades, such as the F strain and the 17+ strain. These strains have undergone mutations and are adapted to infecting and killing cells under artificial culture conditions in the laboratory, but their adaptability to clinical cancer tissues cannot be guaranteed. For example, laboratory strains are passaged in rapidly dividing cell lines, where the number of cells basically doubles every two days, while the division rate of tumor cells in patients is extremely slow, possibly taking one or two months to double. In addition, in the laboratory, viruses are cultured and amplified in two-dimensional monolayer cells, while in reality, viruses need to replicate and spread in three-dimensional spherical tumor tissue. These critical differences between clinical applications and laboratory conditions mean that relying on laboratory strains will bring inherent defects to the development and application of oncolytic viruses. And therefore, clinical strains isolated directly from clinical samples have gradually begun to be used in the development of oncolytic viruses. For example, T-Vec was developed using the clinical isolate JS-1.

However, clinical strains also have their limitations. For example, directly isolated clinical strains are formed through long-term evolution in normal human tissues and cells, especially nerve cells. While they are adapted to cell killing in 3D tissues, they may not have optimal killing ability against cancer cells. Moreover, there is diversity among different clinical strains, and a randomly isolated single clinical strain may not be suitable for oncolytic virus development. And therefore, it is necessary to isolate and screen clinical strains to ensure that excellent clinical strains are obtained for subsequent oncolytic virus development.

The HSV genome is extremely large, nearly 150kb, and later modifications only involve a maximum of 10kb. This means that in oncolytic virus development, oncolytic properties can only be altered and enhanced by modifying a small subset of genes, which constitute less than 10% of the total genome, and the remaining 90% of the HSV genes cannot be modified. These unmodified gene groups determine a significant portion of the oncolytic properties, and this "innate oncolytic property" depends on the chosen original virus strain. And therefore, the selection of the starting HSV strain is one of the most crucial steps in oncolytic virus development.

### SUMMARY OF THE INVENTION

The strain(s) involved in the present disclosure is designed to overcome the limitations of existing laboratory strains and conventional clinical strains. The present disclosure provides a strain with the strongest cancer cell killing ability obtained through screening multiple clinical strains, and an oncolytic-enhanced HSV clinical strain with further enhanced oncolytic properties obtained through methods such as directional evolution in cancer cells.

The present disclosure relates not only to the virus strain, but also to reverse genetic systems constructed based on the virus strain (including systems obtained using BAC technology and homologous recombination technology), as well as oncolytic viruses, vaccine strains, and vaccine vectors engineered from the virus strain.

In some aspects of the present disclosure, an isolated HSV-1 clinical virus strain is provided.

In some embodiments, the present disclosure provides an isolated HSV-1 clinical virus strain designated as C1-0803-1-1-1, deposited at China Center for Type Culture Collection (CCTCC) under the accession no. CCTCC NO: V202351.

In some aspects of the present disclosure, an isolated HSV-1 clinical viral evolution strain is provided.

In some embodiments, the clinical viral evolution strain is obtained through the directed evolution of the clinical virus strain of the present disclosure in cancer cells. In some embodiments, the clinical virus strain is designated as C1-0803-1-1-1, and deposited at CCTCC under accession no. CCTCC NO: V 202351. In some embodiments, the present disclosure provides an HSV-1 clinical viral evolution strain designated as C1-31110, and deposited at CCTCC under accession no. CCTCC NO: V202335.

In some embodiments, the viral evolution strain of the present disclosure is isolated, purified, and amplified. In some embodiments, obtaining the viral evolution strain comprises subjecting a clinical virus strain to successive passages (e.g., 3-10 passages, such as 5 passages); screening the successively passaged virus strain based on viral plaque phenotypes after Vero cells infected with virus; isolating a virus strain that produces a single, larger viral plaque than other virus strains; optionally, purifying the isolated virus strain.

In some aspects of the present disclosure, provided is an infectious clone that comprises a viral genome derived from clinical virus strains or viral evolution strains of the present disclosure, and a bacterial artificial chromosome (BAC) cloning vector backbone sequence with the viral genome inserted therein.

In some embodiments, the infectious clone of the present disclosure comprises a viral genomic DNA into which a BAC cloning vector backbone sequence (e.g. pBeloBAC11-CMV-eGFP backbone sequence) is inserted (e.g., the insertion site is viral genome UL37/UL38).

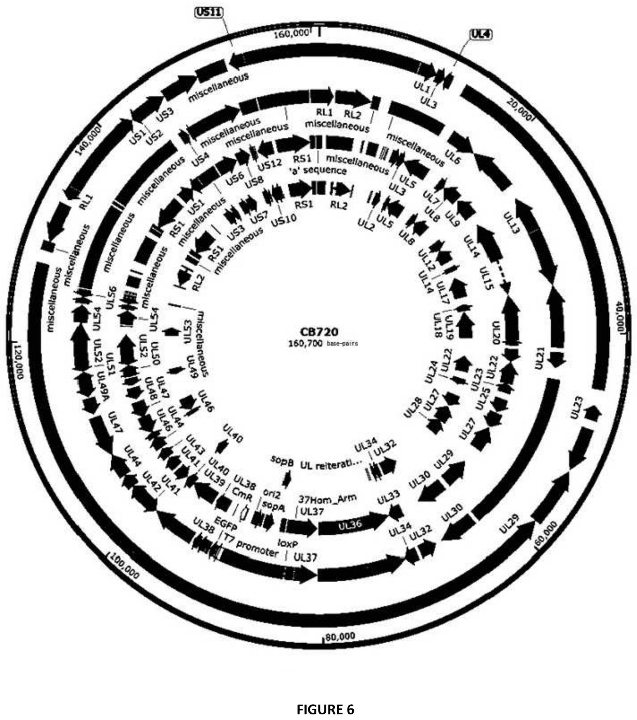

In some embodiments, a plasmid map of the infectious clone of the present disclosure is shown in Figure 6. In some embodiments, the present disclosure provides an infectious clone designated as CB720, deposited at CCTCC under accession no. CCTCC NO: V 202352.

In some aspects of the present disclosure, derived virus strains derived from clinical virus strains, viral evolution strains, or infectious clones of the present disclosure are provided, which are obtained by genetically modifying said clinical virus strains, viral evolution strains, or infectious clones.

In some embodiments, the genetic modification to clinical virus strains or viral evolution strains or infectious clones includes, but is not limited to, one or more selected from the group consisting of (1) reverse genetic modification (e.g., modifications to the viral genome in infectious clones); (2) homologous recombination; and (3) Cre/LoxP site-specific recombination system modification.

In some embodiments, the genetic modification to clinical virus strains, viral evolution strains, or infectious clones includes, but is not limited to, one or more selected from the group consisting of:
Deleting non-essential genes, such as deleting ICP34.5, ICP6, ICP47, functional glycoprotein H encoding gene, functional thymkinase encoding gene; modifying the promoters of essential genes by replacing them with tumor-specific promoters, enabling the virus to replicate exclusively in cancer cells; engineering structural proteins of virus, for example, gB, gD, gH, *etc.,* by inserting domains targeting tumor-associated antigens (TAAs), (e.g., scFvs, VHH, or other domains with specific binding capabilities), enabling the virus to specifically bind and infect TAA-expressing cancer cells; and/or
Introducing heterologous genes that improve immune response and/or tumor suppression, such as genes encoding immunostimulatory polypeptides (e.g., GM-CSF, cytokines or chemokines (CCL5, CCL20, CCL21), RNATES, B7.1, B7.2, IL-12, IL-15, HSP70), genes encoding prodrug-activating proteins (e.g., nitroreductase, cytochrome p450), genes encoding tumor suppressor peptides or tumor suppressor proteins (e.g., p53, TRAIL, anti-PD-1 antibody, endostatin), bispecific antibodies, or immune-killing cell engagers (e.g., T-cell engagers, NK -cell engagers); and/or
Introducing heterologous genes that improve viral replication and spread within tumors or modify the tumor microenvironment, such as ECM degradation genes (hyaluronidase).

In some aspects of the present disclosure, provided is a product that comprises clinical virus strains, viral evolution strains, infectious clones, and/or derived virus strains of the present disclosure.

In some embodiments, the products of the present disclosure are for use as pharmaceutical or viral vector vaccines for tumor prevention and/or treatment, materials for pharmaceutical or vaccine production and/or research and development, and/or exogenous gene vectors.

In some aspects of the present disclosure, provided is the use of the clinical virus strains, viral evolution strains, infectious clones and/or derived virus strains of the present disclosure in the preparation of drugs or viral vector vaccines for tumor prevention and/or treatment or materials for pharmaceutical or vaccine production and/or research and development.

In some aspects of the present disclosure, a method for tumor prevention and/or treatment is provided, wherein the method comprises administering to subjects in need of a prophylactically and/or therapeutically effective amount of clinical virus strains, viral evolution strains, infectious clones and /or derived virus strains of the present disclosure.

In some aspects of the present disclosure, a method for preparing an antitumor directed evolution HSV-1 virus strain is provided, the method comprises screening the HSV-1 clinical virus strains of the present disclosure for antitumor directed evolution.

In some aspects of the present disclosure, a method for preparing an infectious clone is provided, the method comprises providing a viral genome of the HSV-1 clinical virus strains and/or viral evolution strain of the present disclosure, and inserting it into a bacterial artificial chromosome (BAC) cloning vector backbone.

In some aspects of the present disclosure, a method for preparing recombinant viruses is also provided, which comprises further recombinant modification of the HSV-1 clinical virus strains, viral evolution strain and /or infectious clone of the present disclosure.

In some embodiments of the present disclosure, the tumor is selected from solid tumors or non-solid tumors. For example, tumors include, but are not limited to, breast cancer, liver cancer, pharyngeal cancer, lung cancer, and malignant glioma.

Those skilled in the art may arbitrarily combine the above technical solutions and technical features without departing from the inventive concept and protection scope of the present invention. Other aspects of the present invention are obvious to those skilled in the art based on the disclosure of present application.

### DESCRIPTION OF FIGURES

The present invention will be further illustrated below in conjunction with the accompanying drawings, and these drawings are only for illustrating the embodiments of the present invention rather than limiting the scope of the present invention.
**Figure 1** shows the infection of the isolated clinical virus strain C1-0803-1-1-1 in Vero cells:
   Figure 1A: Photo of the cytopathic effect (CPE) of Vero cells infected with C1-0803-1-1-1 virus (10× objective; Nikon camera).
   Figure 1B: Photo of viral plaques in Vero cells infected with C1-0803-1-1-1 virus (GelDoc Imaging System).
**Figure 2** shows the assessment results of the killing ability of the clinical strains:
   Figure 2A: Comparison of the 72-hour killing ability of isolated clinical strains C1-0803-1-1-1, C1-0803-4-1-2, C1-0809-1-1-1, C1-0809-4-1-1 and commercially available strain HSV-1 VR-733 (ATC) against human breast cancer cell line MCF7;
   Figure 2B: Comparison of the 48-hour killing ability of isolated clinical strains C1-0803-1-1-1, C1-0803-4-1-2, C1-0809-1-1-1, C1-0809-4-1-1 and commercially available strain HSV-1 VR-733 (ATC) against human hepatocellular carcinoma cell line Hep3B.
   In the figures, a statistically significance on the killing ability between the clinical strain C1-0803-1-1-1 and the commercially available strain HSV-1 VR-733 (ATCC) was evaluated by t-test (*, p<0.05; **, p<0.01; ***, p<0.005; ****, p<0.001).
**Figure 3** shows the infection of the strain selected during directed evolution (C1-31110 directed evolution strain) in Vero cells:
   Figure 3A: Photo of the cytopathic effect (CPE) of Vero cells infected with C1-31110 virus (10× objective; Nikon camera).
   Figure 3B: Photo of viral plaques in Vero cells infected with C1-31110 virus (GelDoc Imaging System).
**Figure 4** shows the assessment results of the killing ability of the evolved strains:
   Figure 4A: Comparison of the 48-hour killing ability of isolated evolved strains C1-31110, C1-51110, clinical strain C1-0803-1-1-1 and commercially available strain HSV-1 VR-733 against human pharyngeal squamous cell carcinoma cell line FADU.
   Figure 4B: Comparison of the 48-hour killing ability of isolated evolved strains C1-31110, C1-51110, clinical strain C1-0803-1-1-1 and commercially available strain HSV-1 VR-733 against human non-small cell lung cancer cell line NCI-H358.
   Figure 4C: Comparison of the 48-hour killing ability of isolated evolved strains C1-31110, C1-51110, clinical strain C1-0803-1-1-1 and commercially available strain HSV-1 VR-733 against human embryonic lung fibroblast cell line MRC5.
   In the figures, a statistically significance on the killing ability between the evolved strain C1-31110 and the commercially available strain HSV-1 VR733 was evaluated by t-test (ns, no significant difference; *, p<0.05; **, p<0.01; ***, p<0.005; ****, p<0.001).
**Figure 5** shows the killing ability test of the evolved strain in 3D cells:
   Figure 5A: Comparison of the 48-hour killing ability of isolated evolved strain C1-31110, clinical strain C1-0803-1-1-1, and commercially available strain HSV-1 VR-733 against 3D cultured malignant glioma cells U87-MG at MOI=0.5, 1, 3, or 5. Numerically labeled grid and its left and right grids represent one group under the same MOI, with three replicates per MOI. The figure shows a photo panel of 3D spheroid images captured by a high-content imaging system (PerkinElmer Operetta CLS).
   Figure 5B: Comparison of 48-hour spheroid volume of 3D-cultured malignant glioma cells U87-MG by isolated evolved strain C1-31110, clinical strain C1-0803-1-1-1, and commercially available strain HSV-1 VR-733 at MOI=0.5, 1, 3, or 5.
   In the figures, a statistically significance was evaluated by t-test (ns, no significant difference; *, p<0.05; **, p<0.01; ***, p<0.005; ****, p<0.001).
**Figure 6** shows the plasmid map of the BAC-based infectious clones of the evolved strain, specifically the CB720 virus strains map.

### DEPOSITARY INFORMATION

The following biological materials in the present disclosure have been deposited with an international depositary authority body for biological materials recognized by the China Intellectual Property Administration (CNIPA). The specific depositary information is as follows.

| | |
|---|---|
| Biological material sample name | Herpes simplex virus (HSV) C1-0803-1-1-1 |
| Classification nomenclature of biological materials (Latin name) | *Herpes simplex virus* |
| Name of depositary authority | China Center for Type Culture Collection (CCTCC) |
| Address of the depositary authority | Wuhan University, Wuhan, China (No. 299 Bayi Road, Wuchang District, Wuhan City, Hubei Province) |
| Deposit date | 2023.5.31 |
| Accession no. | CCTCC NO: V202351 |
| Status | Alive |
| Biological material sample name | Herpes simplex virus HSV C1-31110 |
| Classification nomenclature of biological materials (Latin name) | *Herpes simplex virus* |
| Name of depositary authority | China Center for Type Culture Collection (CCTCC) |
| Address of the depositary authority | Wuhan University, Wuhan, China (No. 299 Bayi Road, Wuchang District, Wuhan City, Hubei Province) |
| Deposit date | 2023.5.6 |
| Accession no. | CCTCC NO: V202335 |
| Status | Alive |
| Biological material sample name | Herpes simplex virus HSV- BAC infectious clone CB720 |
| Classification nomenclature of biological materials (Latin name) | HSV-BAC *(Herpes simplex virus-bacterial artificial chromosome*) |
| Name of depositary authority | China Center for Type Culture Collection (CCTCC) |
| Address of the depositary authority | Wuhan University, Wuhan, China (No. 299 Bayi Road, Wuchang District, Wuhan City, Hubei Province) |
| Deposit date | 2023.5.31 |
| Accession no. | CCTCC NO: V202352 |
| Status | Alive |

### DETAILED DESCRIPTION

Through extensive and in-depth research, the present applicant has isolated HSV-1 clinical strains from clinical samples of HSV-1-infected patients. By screening viral plaques of these clinical strains, specific clinical strains with high tumor cell-killing activity were obtained. Further, directed evolution strains with superior oncolytic properties were obtained by successively passaging selected strains and screening for anti-tumor activity. Furthermore, using reverse genetics technology, infectious clones stably carrying the genomic information of the oncolytic virus were also obtained in the present disclosure. And therefore, the present disclosure provides a powerful new material for the development of HSV-based oncolytic viruses, with broad and excellent prospects in tumor prevention and/or treatment, as well as related drug development.

In some embodiments of the present disclosure, the HSV-1 clinical strain and directed-evolution isolate of the present disclosure are used to prepare oncolytic viruses. In some embodiments of the present disclosure, the HSV-1 directed-evolution clinical isolate of the present disclosure is used to prepare therapeutic or prophylactic drugs or vaccines. In some embodiments of the present disclosure, the HSV-1 directed-evolution clinical isolate of the present disclosure is used to prepare vaccine vectors.

In some embodiments, the present disclosure includes an oncolytic virus comprising the HSV-1 clinical strain or directed-evolution isolate of the present disclosure. In some embodiments, the present disclosure includes an oncolytic virus engineered from the HSV-1 directed-evolution clinical isolate of the present disclosure. In some embodiments, the present disclosure includes a therapeutic or prophylactic HSV-1 vaccine comprising the HSV-1 directed-evolution clinical isolate of the present disclosure. In some embodiments, the present disclosure includes a therapeutic or prophylactic HSV-1 vaccine engineered from the HSV-1 directed-evolution clinical isolate of the present disclosure. In some embodiments, the present disclosure includes a pharmaceutical composition comprising an oncolytic virus engineered from the HSV-1 directed-evolution clinical isolate of the present disclosure. In some embodiments, the present disclosure includes a pharmaceutical composition comprising a vaccine strain engineered from the HSV-1 directed-evolution clinical isolate of the present disclosure. In some embodiments, the present disclosure includes a pharmaceutical composition comprising a vaccine vector engineered from the HSV-1 directed-evolution clinical isolate of the present disclosure. In some embodiments, the present disclosure includes a pharmaceutical composition comprising a vaccine engineered from the HSV-1 directed-evolution clinical isolate of the present disclosure.

In other embodiments, the present disclosure includes a reverse genetic system constructed based on the HSV-1 directed-evolution clinical isolate of the present disclosure. In some embodiments, the present disclosure includes constructing infectious clones of the HSV-1 directed-evolution clinical isolate of the present disclosure based on BAC and homologous recombination technology. In some embodiments, the exogenous sequence is not lost in multiple successive passages of the BAC-based infectious clone of the HSV-1 directed-evolution clinical isolate.

In some embodiments, the HSV-1 directed-evolution clinical isolate of the present disclosure exhibits a giant plaque phenotype and cell membrane fusion capability. In some embodiments, compared with commercially available strains, the HSV-1 directed-evolution clinical isolate of the present disclosure shows significantly enhanced tumor cell killing ability at the same MOI. In some embodiments, compared with commercially available strains, the HSV-1 directed-evolution clinical isolate of the present disclosure can effectively kill tumor cells in 3D cell spheres that are more similar to the *in vivo* tumor structure.

All numerical ranges provided herein are intended to clearly include all values falling between the range endpoints and the numerical ranges therebetween. Features mentioned in the present application or in the embodiments may be combined. All features disclosed in the present application may be used in combination with any composition form, and each feature disclosed in the present application may be replaced by any alternative feature that can provide the same, equal or similar purpose. Unless otherwise specified, the disclosed features are only general examples of equal or similar features.

As used herein, "comprising", "having", or "including" encompasses "consisting of", "consisting essentially of", and "comprised of"; "consisting essentially of" "substantially consisting of" and "consisted of" are sub-concepts of "comprising", "having", or "including".

### Isolated HSV-1 clinical virus strains and their evolution strains

In some aspects of the present disclosure, an isolated HSV-1 clinical oncolytic virus strain is provided.

In some embodiments, HSV-1 virus is obtained by isolating herpes samples from individuals infected with herpes simplex virus, for example, by isolating the virus from exudate obtained from blistered tissue of the lips and face of patients with oral herpes.

In some embodiments, the virus is subjected to plaque screening, and the resulting high-titer strains are isolated, purified, amplified, and identified. In some embodiments, compared to commercially available strains (e.g., HSV-1 VR733), the selected clinical strains exhibit significantly superior tumor cell killing ability against a variety of tumor cells.

In some embodiments, the isolated HSV-1 clinical oncolytic virus strain is designated as C1-0803-1-1-1, which has a viral titer on Vero cells that is nearly twice that of the commercially available HSV-1 strain VR733, and exhibits a significantly enhanced tumor cell killing ability against a variety of tumor cell lines.

In some aspects of the present disclosure, an isolated HSV-1 clinical viral evolution strain is provided. In some embodiments, it is obtained through the directed evolution of clinical virus strains of the present disclosure in cancer cells.

In some embodiments, obtaining the evolved strain of the present disclosure comprises subjecting the clinical oncolytic virus strains of the present disclosure to successive passages (e.g., 3-20 passages, such as 10 passages, 8 passages, 5 passages); screening the virus strains based on the viral plaque phenotype after infecting Vero cells with the virus, selecting a virus strains that produces single, larger viral plaques than other virus strains; and isolating and purifying the selected virus strains.

Through directed evolution as described in the present disclosure, a specific HSV-1 clinical virus strain designated as C1-31110 was obtained, which exhibits significantly superior tumor cell killing ability against a variety of tumor cells (even at MOI as low as 0.01). Furthermore, the directed-evolution strain also demonstrates highly efficient killing activity against 3D tumor cells.

### Reverse genetic system

In some aspects of the present disclosure, a reverse genetic system obtained through reverse genetics technology is also provided. As used herein, the term "reverse genetic" refers to the process of rescuing live viruses or virus-like materials from viral genetic material in cultured cells or susceptible hosts. The genetic material capable of rescuing viruses is called an "infectious clone," which is generally a bacterial plasmid containing a copy of the entire viral genome cDNA, making the cDNA itself or the RNA transcribed from the cDNA *in vitro* infectious. The viral reverse genetic system, by directionally modifying the viral genome sequence and detecting the phenotype of the rescued artificially modified virus, can effectively study the viral gene structure, function, and virus-host interactions *in vivo.* Through reverse genetic systems and techniques, various modifications or alterations can be made to the viral genome at the DNA level, and the effects of these gene manipulations can be evaluated by the phenotypic changes of the rescued virus. This allows for research on viral genome expression regulation, molecular mechanisms of viral pathogenesis, and the acquisition of attenuated strains for the development of novel vaccines.

The infectious clone of the present disclosure may comprise a viral genome derived from clinical virus strains or viral evolution strains of the present disclosure, and a bacterial artificial chromosome (BAC) cloning vector backbone with the viral genome inserted therein. For example, the infectious clone of the present disclosure comprises a viral genomic DNA (e.g., the insertion site is viral genome UL37/UL38) into which a BAC cloning vector backbone sequence (e.g., pBeloBAC11-CMV-eGFP backbone sequence) is inserted.

In some embodiments, a plasmid map of the infectious clone of the present disclosure may be as shown in Figure 6. In some embodiments, the present disclosure provides an infectious clone designated as CB720, whose loaded foreign gene can be stably present in the genome.

### Further genetic modifications to clinical virus strains, evolution strains, and infectious clones

The clinical strains, evolution strains, and infectious clones of the present disclosure may comprise further genetic modifications, or may be subject to further genetic modifications. For example, one or more genetic modifications selected from the following group may be applied to the clinical virus strains, viral evolution strains, or infectious clones: (1) reverse genetic modification (e.g., modification of the viral genome of the infectious clone); (2) homologous recombination; and (3) Cre/LoxP site-specific recombination system modification.

In some embodiments, the genetic modification includes, but is not limited to, one or more selected from the group consisting of deleting non-essential genes, such as deleting ICP34.5, ICP6, ICP47, functional glycoprotein H encoding gene, functional thymkinase encoding gene; modifying the promoters of essential genes by replacing them with tumor-specific promoters, enabling the virus to replicate exclusively in cancer cells; engineering viral structural proteins, for example, gB, gD, gH, and etc., by inserting domains targeting tumor-associated antigens (TAAs), (e.g., scFvs, VHH, or other domains with specific binding capabilities), enabling the virus to specifically bind and infect TAA-expressing cancer cells; and/or introducing heterologous genes that improve immune response and/or tumor suppression, such as genes encoding immunostimulatory polypeptides (e.g., GM-CSF, cytokines or chemokines (CCL5, CCL20, CCL21), RNATES, B7.1, B7.2, IL-12, IL-15, HSP70), genes encoding prodrug-activating proteins (e.g., nitroreductase, cytochrome p450), genes encoding tumor suppressor peptides or tumor suppressor proteins (e.g., p53, TRAIL, anti-PD-1 antibody, endostatin), bispecific antibodies, or immune-killing cell engagers (e.g., T-cell engagers, NK -cell engagers); and/or introducing heterologous genes that improve viral replication and spread within tumors or modify the tumor microenvironment, such as ECM degradation genes (hyaluronidase).

As used herein, "deletion" or "delete" refers to the partial or complete absence of a nucleotide sequence. Deletion of specific fragments in nucleotide sequences can be achieved using conventional techniques in the art, such as those described in Molecular Cloning: A Laboratory Manual (3rd edition, New York: Cold Spring Harbor Laboratory Press, 1989). In the present disclosure, the deletion of ICP34.5, ICP6, and ICP47 in the viral vector enhances the antitumor specificity and oncolytic ability of the recombinant virus.

Recombinant viruses containing exogenous target genes can be constructed by inserting genes encoding desired proteins, polypeptides, or fragments thereof into viruses or viral vectors. Insertion of exogenous target genes into the viral genome can also be performed using well-known methods described in the references (Graham FL et al., Proc. Natl. Acad. Sci. USA 91:8802-8806 (1994), Miyake S. et al., Proc. Natl. Acad. Sci. USA 93:1320-1324 (1996), etc.).

In some embodiments, the exogenous target gene may further comprise encoding sequences for one or more antitumor molecules and/or antitumor synergistic molecules. In some embodiments, one or more other antitumor molecules and/or antitumor synergistic molecules include, but are not limited to encoding sequences for immune checkpoint inhibitors (e.g., PD1, PDL1, CTLA-4, LAG-3, TIM-3, TIGIT, VISTA), encoding sequences for cytokines (e.g., IL-5, IL-2, IL-6, IL-24, GM-CSF, TNF- α, IFN-γ), and suicide genes (e.g., multi-substrate deoxynucleoside kinase Dm-DNK, thymocyte kinase (TK), cytosine deaminase (CD), and tumor necrosis factor-associated apoptosis ligand (TRAIL)), etc.

### Products and methods

The present disclosure also provides a product (e.g., a pharmaceutical product or kit) containing an effective amount of the HSV-1 clinical strain, evolution strain, and /or infectious clone of the present disclosure, and a pharmaceutically or immunologically acceptable carrier. As used herein, the terms "active substance" and "active substance of the present disclosure" are used interchangeably to refer to the HSV-1 clinical strain, evolution strain, infectious clone, and/or recombinant virus of the present disclosure.

In a preferred embodiment, the product can be used to prevent or treat tumors. Tumors can be solid tumors or non-solid tumors, including but not limited to bladder cancer, liver cancer, lung cancer, breast cancer, melanoma, ovarian cancer, prostate cancer, kidney cancer, intestinal cancer, head and neck cancer, skin cancer, pancreatic cancer, colorectal cancer, squamous cell carcinoma, mesothelioma; hematologic malignancies (such as leukemia), and nervous system tumors (such as glioblastoma, neuroblastoma).

As used herein, the term "comprise" or "include" may also include the term "contain", "consist essentially of" and "consist of". As used herein, the term "pharmaceutically acceptable" ingredient(s) refers to substances that are suitable for use in humans and/or animals without causing excessive adverse side effects (such as toxicity, irritation, and allergic reactions), that is, they have a reasonable benefit/risk ratio. As used herein, the term "effective amount" refers to an amount that can produce a function or activity in humans and/or animals and can be accepted by humans and/or animals.

As used herein, the term "pharmaceutically acceptable carrier" refers to a carrier for the administration of a therapeutic agent, including various excipients and diluents. The term refers to pharmaceutical carriers that are not essential active ingredients themselves and are not excessively toxic after administration. Suitable carriers are well known to those skilled in the art. A full discussion of pharmaceutically acceptable excipients can be found in "Remington's Pharmaceutical Sciences" (Mack Pub. Co., N.J., 1991).

The pharmaceutically acceptable carrier in the composition may comprise a liquid such as water, saline, glycerol and ethanol. In addition, auxiliary substances may also be present in these carriers, such as fillers, disintegrants, lubricants, glidants, effervescent agents, wetting agents or emulsifiers, flavoring agents, pH buffer substances, etc. Generally, these substances can be formulated in a non-toxic, inert and pharmaceutically acceptable aqueous medium, wherein the pH is generally about 5-8, preferably, the pH is about 6-8.

The active substance in the composition of the present disclosure comprises 0.001 to 99.9 wt% of the total weight of the composition; preferably 1 to 95 wt% of the total weight of the composition, more preferably 5 to 90 wt%, and even more preferably 10 to 80 wt%. The rest is a pharmaceutically acceptable carrier and other additives.

As used herein, the term "unit dosage form" refers to a dosage form for which the compositions of the present disclosure are prepared into a single dose for ease of administration, including but not limited to various solid dosage forms (such as tablets), liquid dosage forms, capsules, and sustained-release formulations.

In some embodiments of the present disclosure, the composition is a single dosage form or multiple dosage forms, wherein the content of the active substance is 10⁶-10¹⁴ PFU/dose, for example 10⁷-10¹³ PFU/dose, or 10⁸-10¹² PFU/dose. In some embodiments of this disclosure, the composition is applied daily, every two days, every three days, weekly, every two weeks, every three weeks, monthly, every two months, or every six months, for example, administering 1 to 6 doses.

It should be understood that the effective dose of the active substance used can vary depending on the severity of the condition of the patient being administrated or treated. The specific dosage is determined based on the individual patient's circumstances (e.g., weight, age, physical condition, and desired outcome), within the judgment of a skilled physician.

The medicaments or active substances described herein may be in solid form (such as granules, tablets, lyophilized powders, suppositories, capsules, sublingual lozenges) or liquid form (such as solutions) or other suitable shapes. The routes of administration may include: (1) direct injection, such as intravenous injection or perfusion, intraperitoneal injection, intratumoral injection, intracranial injection, etc.; (2) linking recombinant viruses with transferrin/poly-L-lysine complexes to enhance their biological effects; (3) forming complexes through combining viruses with positively charged lipids; (4) encapsulating recombinant adenoviruses with liposomes; and (5) transfecting viruses into transferring cells.

Furthermore, the compositions of the present disclosure may also comprise other active substances for improving and treating tumors. In some embodiments, the other active substances may include, but are not limited to, one or more of the following: monoclonal antibody drugs, such as rituximab, trastuzumab, bevacizumab, etc.; cytotoxic agents, such as alkylating agents, nitrogen mustard, platinum mixtures, methotrexate, 5-FU, cytarabine, gemcitabine, actinomycin D, daunorubicin, irinotecan, topotecan, hydroxycamptothecin, paclitaxel, docetaxel, vinblastine, norvincristine, podophyllotoxin, homoharringtonine, etc.; biological response modifiers, such as interferon, interleukin, thymosin, etc.

The active substances described in the present disclosure can be used in combination with each other, as well as with other drugs and treatments. For example, recombinant viruses can be combined with one or more therapies selected from the group consisting of immunotherapy, gene therapy, chemotherapy, radiotherapy, and surgery.

### EXAMPLES

The present application will be further described below in conjunction with specific embodiments. It should be understood that these embodiments are only used to illustrate the present application and are not intended to limit the scope of the present application. Those skilled in the art may make appropriate modifications and changes to the present invention, and these modifications and changes are all within the scope of the present invention.

The experimental methods in the following examples where specific conditions are not specified are usually carried out under conventional conditions such as those described in Sambrook et al., Molecular Cloning: A Laboratory Manual (New York: Cold Spring Harbor Laboratory Press, 1989), or under conditions recommended by the manufacturer. Unless otherwise stated, percentages and parts are calculated by weight.

Unless otherwise defined, all technical and scientific terms used herein have the same meanings as those familiar to those skilled in the art. In addition, any methods and materials similar or equivalent to those described herein may be applied to this application. The preferred implementation methods and materials described herein are for demonstration purposes only.

### Example 1. Clinical Sample Collection

1. After cleaning the wounds of volunteers with oral herpes with saline, about 10 µl of tissue exudate from the blisters on the volunteers' lips and face was collected using a sterile swab;
2. The swab was placed into a pre-prepared virus sampling tube, and the tube containing the swab was stored in a -80°C freezer for subsequent virus culture and isolation;
3. After sampling, iodine solution was used to disinfect the volunteers' wounds to reduce the risk of virus infection.

### Example 2. Isolation of Clinical strains

### Small-scale amplification of clinical virus strains

1. Vero cells (African green monkey kidney cells, SAILY BIO) on a T75 flask were digested with trypsin; and after digestion and counting, the Vero cells were plated into a 6-well plate at 1×10⁶ cells per well, and they are incubated overnight in an incubator at 37°C, 5% CO₂;
2. One day after the cell culture, the cells were observed to form a confluent monolayer without gaps; the virus sample tube was retrieved from the -80°C freezer, and thawed in a 37°C water bath;
3. The supernatant from the 6-well plate was aspirated; 4 ml of thawed virus sample was added to each well to infect the cells using a pipette; and the 6-well plate was incubated in an incubator at 37°C, 5% CO₂;
4. The 6-well plate was gently mixed every 15 minutes to ensure that the virus infects Vero cells more evenly;
5. 2 hours later, the virus supernatant was aspirated; 2 ml of DMEM containing 2% inactivated serum was added to each well; and the 6-well plate was incubated in an incubator at 37°C, 5% CO₂;
6. Observing the morphological changes of virus-infected cells, and after most cells were infected, the supernatant and cells were collected into 15 ml centrifuge tubes and stored at -80°C freezer for later use.

### Isolation and purification of clinical virus strains

1. Based on the titer of small-scale amplified clinical virus strains, a gradient dilution of the virus was performed using 2% DMEM to achieve 1, 5, or 10 infectious virus particles per 500 µl of diluted virus solution;
2. Observing the density of Vero cells in the 6-well plate (seeded at 1×10⁶ cells/well one day prior and incubated overnight in an incubator at 37°C, 5% CO₂), and when the Vero cells formed a confluent monolayer without gaps, the cell supernatant was discarded and 500 µl of diluted virus solution was added; and the 6-well plate was incubated in an incubator at 37°C, 5% CO₂;
3. The 6-well plate was gently mixed every 15 minutes to ensure that the virus attached and infected Vero cells more evenly;
4. 1 hour and 15 minutes later, 1 ml of DMEM supplemented with 2% inactivated serum was added to each well; and the 6-well plate was incubated in an incubator at 37°C, 5% CO₂ for another 2 hours;
5. 2 hours later, the virus culture supernatant was discarded, 500 µl DMEM was added to each well to cover the cells, followed by 3 ml methylcellulose to immobilize the virus, and finally the 6-well plate was incubated in an incubator at 37°C, 5% CO₂;
6. 3 days after virus culture, obvious viral CPE can be observed with the naked eye, and 2 ml neutral red was added to each well and incubated in an incubator overnight;
7. The number of viral plaques in each well was counted on a white plate, and the viral plaques from the wells with the fewest viral plaques were picked out. The virus isolation process was repeated 3 to 4 times until only a single viral plaque was found in each well, which indicated that the purification was complete.

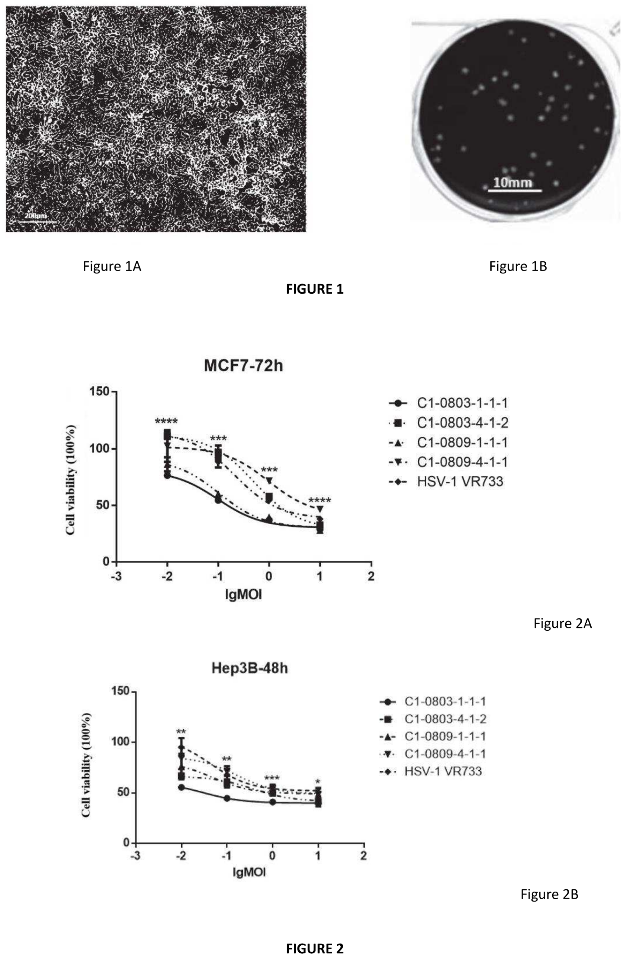

Clinical virus strains C1-0803-1-1-1, C1-0803-4-1-2, C1-0809-1-1-1, and C1-0809-4-1-1 were obtained through the above steps, wherein the viral plaques of the clinical virus strain C1-0803-1-1-1 are shown in Figure 1, and the plaque phenotypes of the other three isolated virus strains are similar to those of C1-0803-1-1-1. Figure 1A shows CPE image of Vero cells infected with C1-0803-1-1-1 (CCTCC NO: V20235 1) virus; and Figure 1B is a viral plaque image of Vero cells infected with C1-0803-1-1-1 virus.

The results showed that a clinical virus strain with infectious and killing effects on Vero cells was obtained by the above methods.

### Example 3. Strain amplification and titration

### Virus Amplification

1. Vero cells on a T75 flask were digested with trypsin; and after digestion and counting, the Vero cells were plated into new T75 flasks at 3×10⁶ cells per flask, and they were incubated overnight in an incubator at 37°C, 5% CO₂;
2. 48 hours after overnight culture, the Vero cells on the T75 flask were confluent monolayer with no gaps between the cells; one T75 flask of cells was digested with trypsin; and the number of cells per flask was counted as a;
3. The Vero cells in T75 flasks were infected with clinical strains C1-0803-1-1-1, C1-0803-4-1-2, C1-0809-1-1-1, C1-0809-4-1-1 or commercial available virus HSV-1 VR-733 (ATCC) at MOI=0.02 PFU/cell; the virus titer was recorded as b (PFU/ml), the required virus volume V(ml) per flask = a*0.02/b, and the final volume of T75 cell infection per flask was 5 ml;
4. The cell supernatant from the T75 cell flask was aspirated and discarded, the Vero cells on the T75 flask were infected with 5 ml 2% FBS DMEM containing the required amount of virus, and the virus-infected T75 cell flasks were incubated in an incubator at 37°C, 5% CO₂;
5. The T75 flasks were gently mixed every 15 minutes to ensure the virus attached and infected the Vero cells evenly;
6. 1 hour and 15 minutes later, the cell supernatant containing virus was discarded, 18 ml of culture medium was added to each T75 flask, and the cell plates were incubated in an incubator at 37°C, 5% CO₂;
7. After culturing overnight in an incubator, the state of virus-infected cells was observed under a microscope; once approximately 100% of the virus exhibited the CPE morphology, the cells were collected using a cell scraper and the cell supernatant containing the cells was collected into a 50 ml centrifuge tube and stored at -80°C freezer for later use.

### Titer determination

1. Based on the estimated virus titer, the virus was serially diluted using 2% DMEM to achieve 30-100 infectious virus particles per 500 µl of virus dilution;
2. The density of the Vero cells in the 6-well plate (plated at 1×10⁶ cells/well one day prior and incubated overnight in an incubator at 37°C, 5% CO₂) was observed; and when the cells formed a confluent monolayer without gaps, the cell supernatant was discarded and 500 µl of virus dilution was added; and the 6-well plate was incubated in an incubator at 37°C, 5% CO₂;
3. The 6-well plate was gently mixed every 15 minutes to ensure that the virus attached and infected the Vero cells more evenly;
4. 1 hour and 15 minutes later, 1 ml of DMEM supplemented with 2% inactivated serum was added to each well; and the 6-well plate was incubated in an incubator at 37°C, 5% CO₂ for another 2 hours;
5. 2 hours later, the virus culture supernatant was discarded, 500 µl DMEM was added to each well to cover the cells, followed by 3 ml methylcellulose to immobilize the virus, and finally the 6-well plate was incubated in an incubator at 37°C, 5% CO₂;
6. 3 days after virus culture, obvious viral CPE can be observed with the naked eye; the virus culture supernatant was discarded, 300 µl of crystal violet staining solution containing fixed formaldehyde was added to each well, and stained at room temperature for 30 minutes;
7. After staining, the crystal violet staining solution was discarded, and the cells were washed three times with PBS until the crystal violet staining solution was completely removed and the viral plaques were clear;
8. The number of viral plaques in each well was counted on a white plate, and the virus titer (PFU/ml) = a*b*1000/500 was calculated based on the virus dilution a and the number of viruses b.

The virus titers of each isolated clinical virus strains (C1-0803-1-1-1, C1-0803-4-1-2, C1-0809-1-1-1, C1-0803-4-1-1) and commercially available HSV-1 VR733 are shown in the table below:

| **Virus strain** | **Titer (PFU/ml)** |
|---|---|
| C1-0803-1-1-1 | 1.15E9 |
| C1-0803-4-1-2 | 5.33E8 |
| C1-0809-1-1-1 | 4.57E8 |
| C1-0803-4-1-1 | 4.10E8 |
| HSV-1 VR733 | 5.95E8 |

The above results demonstrate that through the screening described in the present disclosure, clinical virus strains (i.e., C1-0803-1-1-1, CCTCC NO. V202351) with virus titers comparable to or even nearly twice that of the commercially available HSV-1 were obtained.

### Example 4. Killing Ability Assessment of Clinical Strains

1. Tumor cells, e.g., MCF7 (human breast cancer cells), Hep3B (human liver cancer cells), on T75 flasks were digested with trypsin; and after digestion and counting, cells were plated into 96-well plates at 1×10⁴ cells/100 µl per well, and the plate was incubated in an incubator overnight at 37°C, 5% CO₂;
2. 24 hours after overnight culture, the confluence of tumor cells on the 96-well plate was approximately 90%; the tumor cells in one well were digested with trypsin, and the number of cells was counted as a;
3. Tumor cells in 96-well plates were infected with clinical virus strains obtained using the methods described in Examples 1 and 2, including C1-0803-1-1-1, C1-0803-4-1-2, C1-0809-1-1-1, C1-0809-4-1-1, and HSV-1 VR733 at MOIs of 10, 1, 0.1, or 0.01 PFU/cell, respectively; the virus titer was recorded as b (PFU/ml), the required virus volume V(ml) per well = a*10/b (Triple replicate) (if MOI=10 PFU/cell), and the final volume of cell infection per well in the 96-well plate was 100 µl;
4. 24, 48, and 72 hours after virus infection, 20 µl of CCK8 detection reagent (enhanced CCK-8 kit, Beyotime, C0042) was added to each well, and it was incubated in an incubator at 37°C for approximately 2 hours (the exact incubation time depended on the color change of the culture medium); the OD value of the culture medium supernatant was measured at OD _{450 nm}, and the cell viability was calculated (with blank wells and cell wells set).

The results of the killing ability assessment of the isolated clinical strains C1-0803-1-1-1, C1-0803-4-1-2, C1-0809-1-1-1, C1-0809-4-1-1 and the commercially available strain HSV-1 VR-733 in tumor cells, such as MCF7 and Hep3B, are shown in Figure 2A and Figure 2B, respectively. Compared with the commercially available strain HSV-1 VR733, the clinical strain C1-0803-1-1-1 showed a significantly enhanced tumor cell killing ability at the same MOI.

### Example 5. Directed Evolution

### Successive passages of virus

1. The density of Vero cells in a 6-well plate (plated at 10⁶ cells per well one day in advance and incubated overnight in an incubator at 37°C, 5% CO₂) was observed; once there were no gaps between the cells, the cell supernatant was discarded; 1 µl of C1-0803-1-1-1 virus and 500 µl of 2% FBS DMEM were added to one well of the 6-well plate; and the 6-well plate was further incubated in an incubator at 37°C, 5% CO₂;
2. The 6-well plate was gently mixed every 15 minutes to ensure that the virus attached and infected the Vero cells more evenly;
3. 1 hour and 15 minutes later, 1 ml of DMEM containing 2% inactivated serum was added to each well, the 6-well plate was further incubated in an incubator at 37°C, 5% CO₂ for 2 hours; then the supernatant was discarded, 2 ml of DMEM containing 2% inactivated serum was added, and further incubated in an incubator at 37°C, 5% CO₂;
4. The virus infection status was observed, and the virus was collected when the virus infection was 100% (approximately 48 hours);
5. After repeatedly freezing and thawing the collected virus at -80 °C and 37 °C three times, it was centrifuged at 3000 rpm and 4°C for 10 min; then the supernatant was collected, and stored in a -80°C freezer for the next round of virus infection. The above steps were repeated to blindly passage the virus five times, and finally virus screening and isolation were performed.

### Evolution strain screening and isolation

1. The P5 generation virus that had undergone 5 blind passages was taken and purified by plaque; the virus was serially diluted and Vero cells in 6-well plates were infected with 500 µl of the virus dilution (plated at 10⁶ cells per well one day in advance and incubated overnight in an incubator at 37°C, 5% CO₂) to achieve 1, 5, 10 or 100 infectious virus particles per well; the plate was incubated in an incubator at 37°C, 5% CO₂;
2. The 6-well plate was gently mixed every 15 minutes to ensure that the virus attached and infected the Vero cells more evenly;
3. 1 hour and 15 minutes later, 1 ml of DMEM supplemented with 2% inactivated serum was added to each well; and the 6-well plate was incubated in an incubator at 37°C, 5% CO₂ for another 2 hours;
4. 2 hours later, the virus culture supernatant was discarded, 500 µl DMEM was added to each well to cover the cells, followed by 3 ml methylcellulose to immobilize the virus, and finally the 6-well plate was incubated in an incubator at 37°C, 5% CO₂;
5. After culturing the virus for 48 hours, 2 ml of neutral red was added to each well for overnight staining; the virus staining was observed the next day and viruses with only a single viral plaque in each well and a relatively large plaque phenotype were selected. The above steps were repeated until a virus strain with a giant plaque phenotype and cell membrane fusion ability was isolated.

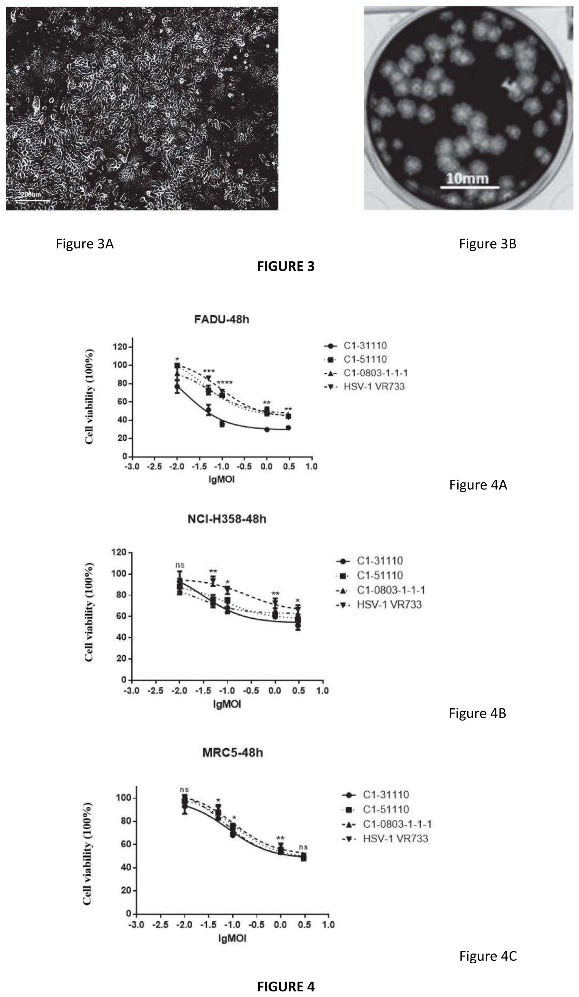

Directed evolution strains C1-31110 and C1-51110 were obtained, with the viral plaques of directed evolution strain C1-31110 shown in Figure 3. This result indicates that directed evolution yielded virus C1-31110 (CCTCC NO: V202335), which exhibited a giant plaque phenotype and cell membrane fusion capability.

### Example 6. Amplification and Titration of Evolution strains

### Virus Amplification

1. Vero cells on a T75 flask were digested with trypsin; and after digestion and counting, the Vero cells were plated into new T75 flasks at 3×10⁶ cells per flask, and it was incubated overnight in an incubator at 37°C, 5% CO₂;
2. 48 hours after overnight culture, the Vero cells in the T75 flask were confluent monolayer with no gaps between the cells; one T75 flask of cells was digested with trypsin; and the number of cells per flask was counted as a;
3. Vero cells on a T75 plate were infected with viruses at an MOI of 0.02 PFU/cell, and the virus titer was recorded as b. (PFU/ml), then the required virus volume V(ml) per plate = a*0.02/b, and the final volume of T75 cell infection per plate was 5 ml;
4. The cell supernatant from the T75 cell flask was aspirated and discarded, the Vero cells on the T75 flask were infected with 5 ml 2% FBS DMEM containing the required amount of virus, and the virus-infected T75 cell flask was incubated in an incubator at 37°C, 5% CO₂;
5. The T75 flask was gently mixed every 15 minutes to ensure that the virus attached and infected the Vero cells evenly;
6. 1 hour and 15 minutes later, the cell supernatant containing virus was discarded, 18 ml of culture medium was added to each T75 plate, and the cell plate was incubated in an incubator at 37°C, 5% CO₂;
7. After culturing overnight in an incubator, the state of virus-infected cells was observed under a microscope; once approximately 100% of the virus exhibited the CPE morphology, the cells were collected using a cell scraper and the cell supernatant containing the cells was collected into a 50 ml centrifuge tube and stored at -80°C freezer for later use.

### Titer determination

1. Based on the estimated virus titer, the virus was serially diluted using 2% DMEM to achieve 30-100 infectious virus particles per 500 µl of virus dilution;
2. The density of Vero cells in the 6-well plate (plated at 1×10⁶ cells/well one day prior and incubated overnight in an incubator at 37°C, 5% CO₂) was observed; and when cells formed a confluent monolayer without gaps, the cell supernatant was discarded and 500 µl of diluted virus solution was added; and the 6-well plate was incubated in an incubator at 37°C, 5% CO₂;
3. The 6-well plate was gently mixed every 15 minutes to ensure that the virus attached and infected the Vero cells more evenly;
4. 1 hour and 15 minutes later, 1 ml of DMEM supplemented with 2% inactivated serum was added to each well; and the 6-well plate was incubated in an incubator at 37°C, 5% CO₂ for another 2 hours;
5. 2 hours later, the virus culture supernatant was discarded, 500 µl DMEM was added to each well to cover the cells, followed by 3 ml methylcellulose to immobilize the virus, and finally the 6-well plate was incubated in an incubator at 37°C, 5% CO₂;
6. 3 days after virus culture, obvious viral CPE can be observed with the naked eye; the virus culture supernatant was discarded, 300 µl of crystal violet staining solution containing fixed formaldehyde was added to each well, and stained at room temperature for 30 minutes;
7. After staining, the crystal violet staining solution was discarded, and the cells were washed three times with PBS until the crystal violet staining solution was completely removed and the viral plaques were clear;
8. The number of viral plaques in each well was counted on a white plate, and the virus titer (PFU/ml) = a*b*1000/500 was calculated based on the virus dilution a and the number of viruses b.

The results showed that the virus titer of the directed evolution strain C1-31110 was 2.11E8 PFU/ml; and the virus titer of C1-51110 virus was 2.5E9 PFU/ml.

### Example 7. Killing Ability Test of Evolution Strains

1: 1. Tumor cells, e.g., FADU (Human pharyngeal squamous cell carcinoma cells), NCI-H358 (Human non-small cell lung cancer cells), and MRC5 cells on T75 plates were digested with trypsin; and after digestion and counting, tumor cells, e.g., FADU cells and NCI-H358 cells, and MRC5 cells were plated into 96-well plates at 1 x 10⁴ cells/100 µl /well and incubated overnight in an incubator at 37°C, 5 % CO₂;
2. 24 hours after overnight culture, the confluence of tumor cells in the 96-well plate was approximately 90%; the tumor cells in one well were digested with trypsin, and the number of cells was counted as a;
3. Each tumor cells and MRC5 cells in a 96-well plate were infected with virus at MOIs of 3, 1, 0.1, 0.05, or 0.01 PFU/cell, respectively; the virus titer was recorded as b. (PFU/ml), then the required virus volume V(ml) per well = a*10/b (Triple replicate) (If MOI=10 PFU/cell), the final volume of cell infection per 96-well plate was 100 µl;
4. 24, 48, and 72 hours after virus infection, 20 µl of CCK8 detection reagent was added to each well, and it was incubated in an incubator at 37°C for approximately 2 hours (the exact incubation time depended on the color change of the culture medium); the OD value of the culture medium supernatant was measured at OD _{450 nm}, and the cell viability was calculated (with blank wells and cell wells set).

The results of the tumor cell killing ability assessment of the evolved C1-31110 and C1-51110, the clinical strain C1-0803-1-1-1, and the commercially available strain HSV-1 VR-733 in FADU, NCI-H358, and MRC5 cells are shown in Figures 4A, 4B, and 4C, respectively. Compared with the commercially available strain HSV-1 VR733, the evolved C1-31110 strain shows a significantly enhanced tumor cell killing ability at the same MOI, and the tumor cell killing abilities of the evolution strain C1-51110 and the clinical strain C1-0803-1-1-1 are also improved to some extent. However, the overall killing ability of the evolution strain C1-31110 against embryonic lung fibroblasts (MRC5) is not significantly different from that of the commercially available HSV-1.

### Example 8. Killing Ability Test of Evolution Strains in 3D Cells

1. Using 10% DMEM+1%PS complete medium, 6E3 U87-MG cells (malignant glioma cells) and 3E3 MRC5 cells (human embryonic lung fibroblasts, used for co-culturing with cancer cells to form 3D structures) were seeded in each well of a ULA 96-well plate.
2. Cells were centrifuged at 300g for 5 minutes at room temperature, then incubated at 37°C, 5% CO₂ for 48 hours to form spheroids.
3. The spheroid cell count was estimated at 2 × 10⁴ cells, and cells were infected with viruses C1-31110, C1-0803-1-1-1, and HSV-1 VR733 at MOIs of 0.5, 1, 3, or 5 PFU/cell, respectively.
4. High-content imaging system (Perkin Elmer Operetta CLS) was used to capture images of 3D spheroids, and their volumes were calculated.
5. Daily imaging monitored virus infection status, with spheroid volume quantified 48 hours post-infection.

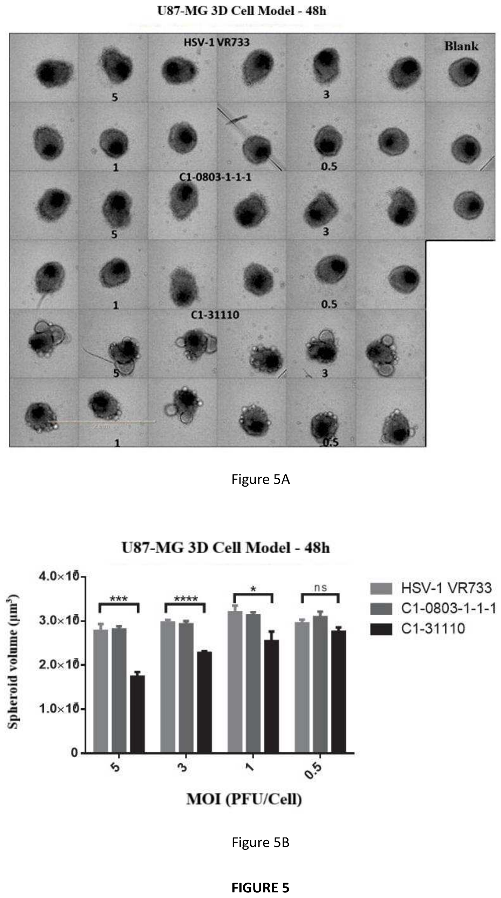

The results are shown in Figure 5. As shown in results, the directed-evolution strain C1-31110 of the present disclosure demonstrated significantly enhanced tumor-killing efficacy in 3D spheroids (which better mimic in vivo tumor structures) compared to HSV-1 VR733 at 48 hours post-infection.

### Example 9. Construction of BAC-Based Infectious Clones for Evolution Strains

### 1. Construction and linearization of repaired donor DNA

pBeloBAC11 plasmid (Fenghui Biotechnology ; ZT178) was used as the cloning vector, the CMV-eGFP expression cassette (template sequence as shown in SEQ ID NO. 1, amplification primer CMV_HomF (SEQ ID NO. 2); bGH_HomR (SEQ ID NO. 3)) was used to replace the original loxp site of the vector and the lacZα inducible expression cassette (vector amplification primer pBAC_HomF (SEQ ID NO. 4); pBAC_Hom R (SEQ ID NO. 5)). InFusion ligation (2 × EasyGeno Recombinant Kit, Tiangen, catalog number. VI201-02) and transformation were performed to obtain pBeloBAC11-CMV-eGFP intermediate vector.

The extracted genomic DNA of the evolution strain was used as a template, two sequences of approximately 1000bp of the UL37 gene (primer 37BAC_loxP_F (SEQ ID NO: 6); 37BAC_PacI_R (SEQ ID NO: 7)) and UL38 gene (primer 38BAC_PacI_F (SEQ ID NO: 8); 38BAC_loxp_R (SEQ ID NO: 9)) flanking either side of the editing target site were amplified using long primers and used as homologous arms; and PacI restriction sites and co-directional loxP sequences were introduced at both ends using primers. The homologous arms at both ends were ligated using overlap PCR to obtain homologous arm sequences with structures such as loxP-UL37Arm-PacI-UL38Arm-loxP.

The homology arm sequences were inserted downstream of the eGFP expression cassette in the intermediate vector (vector amplification primers pBAC-loxP-VF2 (SEQ ID NO. 10); pBAC-loxp-VR2 (SEQ ID NO. 11)) to obtain pBeloBAC11-CMV-eGFP- 37.38 donor clone. After plasmid extraction, it was digested with PacI restriction endonuclease and purified to obtain linearized repair donor DNA containing the BAC backbone.

### 2. Recombining the BAC backbone into the genome of the clinical strain

One day in advance, 293A cells were plated in 24-well plates at 2 x 10⁵ cells/500 µl /well, and 1 µg of linearized BAC backbone repair donor DNA and 1 µg PX330-sgRNA (PX330 plasmid. Fenghui Bio-BR613; sgRNA sequence (SEQ ID NO. 12)) plasmid was transfected into cells using the Lipofectamine 3000 liposome transfection kit (Invitrogen, catalog number L3000-008) . Cells were incubated at 37°C, 5% CO₂ after transfection, and 6 hours later, medium was replaced with fresh medium supplemented with SCR7 (MCE, catalog number HY-107845) (final concentration 10 µM). At 24 hours post-transfection, the evolution strain C1-31110 was infected at a MOI of 0.01, a constant concentration of 10 µM SCR7 was maintained in the culture medium, and incubated in an incubator at 37°C, 5% CO₂ Cells and supernatant were harvested after 24 hours, subjected to three -80/37°C freeze-thaw cycles, generating P₀ recombinant virus sample. This sample contained a large amount of wild-type virus and a very small amount of successfully recombined virus, requiring further screening and purification.

### 3. Coarse screening of recombinant viruses

One day in advance, Vero cells were plated in 6-well plates at 1 x 10⁶ cells /1.5 ml/well. The recombinant virus obtained in the previous round was serially diluted 10-fold and used to infect the Vero cells in 6-well plates. After 2 hours of incubation, the medium was replaced with medium containing 1% methylcellulose, and the plates were incubated in an incubator at 37 °C, 5% CO₂. Approximately 24 hours later, viral plaques with GFP fluorescent signals were selected using a fluorescence microscope to obtain the next-generation virus sample P_{n +1}. Multiple rounds of screening for the recombinant virus in 6-well plates were required until the majority of infected plaques were fluorescent, at which point monoclonal selection of the virus could be performed.

### 4. Monoclonal selection of recombinant viruses

Vero cells were plated in 96-well plates at 8 x 10³ cells/100 µl/well and incubated in an incubator overnight. The coarsely screened and purified recombinant virus sample was serially diluted (2-5-fold) by limit dilution, Vero cells were infected at 100 µl per well, and the plate was incubated in an incubator at 37°C, 5% CO₂. Approximately 24 hours later, fluorescent plaques were observed under a fluorescence microscope and labeled. Culturing continued until the CPE reached 50%, then the virus samples were harvested. During harvesting, viral monoclonal strains were selected from the highest possible dilution groups.

### 5. Identification of recombinant viral monoclonal strains

A small volume (about 50-100 µl) was taken from the total sample to select the recombinant virus monoclonal sample. DNA was extracted using a viral DNA extraction kit, and PCR identification and sequencing were performed on the target site and BAC backbone. Sequence alignment was performed based on the sequencing results. The viral monoclonal sample confirmed to have the pBeloBAC1 1-CMV-eGFP backbone sequence inserted into the UL37/UL38 region of the viral genome was selected as the formal evolution strain, a BAC-based infectious clone, and named CB720 (CCTCC NO. V202352). This clone was ready for amplification, cryopreservation, and use. The plasmid map of the BAC-based infectious clone CB720 of the strain is shown in Figure 6.

### 6. Monoclonal performance assay - Stability of Exogenous Sequence in the Genome

The viral monoclonal strains selected in step 5 were designated as SP₀ (Successive Passage). Gradient infection was performed on pre-coated Vero cells in 6-well plates, and the plates were incubated in a CO₂ incubator for 1-2 days. Wells with a mild infection status (with cytopathic effect CPE approximately 10%-30%, where plaques remained discrete and not confluent) were selected, and the culture supernatant was collected to obtain SPₙ₊₁. This process was repeated to infect new Vero cells for successive passages. During successive passages, the fluorescence of the plaques was observed using a fluorescence microscope, focusing on detecting any loss of fluorescence. No loss of fluorescence was observed in eight successive passages.

100 µL of supernatants from passages SP₀, SP₁, SP₄, and SP₈ were taken, respectively, and viral DNA was extracted using a viral DNA extraction kit. PCR identification was performed targeting the recombination junction regions between the exogenous BAC backbone and the native viral sequences, *i.e*., UL37Arm (primers UL37_iF3 (SEQ ID NO: 13); sopC-iR (SEQ ID NO: 14)) and UL38Arm (primers eGFP_BACinf_F (SEQ ID NO: 15); UL38-iR3 (SEQ ID NO: 16)) fragments, as well as BAC backbone-specific sopB-repE (primers sopB-iF2 (SEQ ID NO: 17); repE-iR1 (SEQ ID NO: 18)) and ChlR+ (primers BAC_seque-iF (SEQ ID NO: 19) and ChlR+_iR3 (SEQ ID NO: 20)) fragments. PCR results confirmed that the exogenous BAC backbone sequence was retained without loss over the eight consecutive passages.

### 7. Functional Testing

Functional testing demonstrated that the BAC-based infectious clone CB720 of the evolution strain can efficiently rescue the virus to produce a recombinant virus with excellent oncolytic activity, and the infectious clone CB720 of the evolution strain has enabled convenient and efficient genetic modification.

All references cited herein are incorporated by reference in their entirety as though each reference was individually incorporated by reference. It should be understood that after reading the contents of this application, those skilled in the art may conduct various changes or modifications to the present application, and these equivalent forms also fall within the scope defined by the claims attached to this application.

Appendix. Sequence numbers, sequence names, and specific sequences of nucleic acid molecules used in each step of the examples:

| **SEQ ID NO:** | **Sequence name** | **Specific sequences** |
|---|---|---|
| **1** | CMV-eGFP DNA | GACATTGATTATTGACTAGTTATTAATAGTAATCAATTACGGGGTCA TTAGTTCATAGCCCATATATGGAGTTCCGCGTTACATAACTTACGGT AAATGGCCCGCCTGGCTGACCGCCCAACGACCCCCGCCCATTGACG TCAATAATGACGTATGTTCCCATAGTAACGCCAATAGGGACTTTCCA TTGACGTCAATGGGTGGAGTATTTACGGTAAACTGCCCACTTGGCA GTACATCAAGTGTATCATATGCCAAGTACGCCCCCTATTGACGTCAA TGACGGTAAATGGCCCGCCTGGCATTATGCCCAGTACATGACCTTAT GGGACTTTCCTACTTGGCAGTACATCTACGTATTAGTCATCGCTATT ACCATGGTGATGCGGTTTTGGCAGTACATCAATGGGCGTGGATAGC GGTTTGACTCACGGGGATTTCCAAGTCTCCACCCCATTGACGTCAAT GGGAGTTTGTTTTGGCACCAAAATCAACGGGACTTTCCAAAATGTC |
| | | GTAACAACTCCGCCCCATTGACGCAAATGGGCGGTAGGCGTGTACG GTGGGAGGTCTATATAAGCAGAGCTCTCTGGCTAACTAGAGAACCC ACTGCTTACTGGCTTATCGAAATTAATACGACTCACTATAGGGAGA CCCAAGCTGGCTAGCGTTTAAACTTAAGCTTGGTACCGAGCTCGGA TCCGCCACCATGGTGAGCAAGGGCGAGGAGCTGTTCACCGGGGTGG TGCCCATCCTGGTCGAGCTGGACGGCGACGTAAACGGCCACAAGTT CAGCGTGTCCGGCGAGGGCGAGGGCGATGCCACCTACGGCAAGCTG ACCCTGAAGTTCATCTGCACCACCGGCAAGCTGCCCGTGCCCTGGC CCACCCTCGTGACCACCCTGACCTACGGCGTGCAGTGCTTCAGCCGC TACCCCGACCACATGAAGCAGCACGACTTCTTCAAGTCCGCCATGC CCGAAGGCTACGTCCAGGAGCGCACCATCTTCTTCAAGGACGACGG CAACTACAAGACCCGCGCCGAGGTGAAGTTCGAGGGCGACACCCTG GTGAACCGCATCGAGCTGAAGGGCATCGACTTCAAGGAGGACGGC AACATCCTGGGGCACAAGCTGGAGTACAACTACAACAGCCACAACG TCTATATCATGGCCGACAAGCAGAAGAACGGCATCAAGGTGAACTT CAAGATCCGCCACAACATCGAGGACGGCAGCGTGCAGCTCGCCGAC CACTACCAGCAGAACACCCCCATCGGCGACGGCCCCGTGCTGCTGC CCGACAACCACTACCTGAGCACCCAGTCCGCCCTGAGCAAAGACCC CAACGAGAAGCGCGATCACATGGTCCTGCTGGAGTTCGTGACCGCC GCCGGGATCACTCTCGGCATGGACGAGCTGTACAAGtgaGCGGCCGC TCGAGTCTAGAGGGCCCGTTTAAACCCGCTGATCAGCCTCGACTGT GCCTTCTAGTTGCCAGCCATCTGTTGTTTGCCCCTCCCCCGTGCCTTC CTTGACCCTGGAAGGTGCCACTCCCACTGTCCTTTCCTAATAAAATG AGGAAATTGCATCGCATTGTCTGAGTAGGTGTCATTCTATTCTGGGG GGTGGGGTGGGGCAGGACAGCAAGGGGGAGGATTGGGAAGACAAT AGCAGGCATGCTGGGGATGCGGTGGGCTCTATGG |
| 2 | CMV_HomF | TGAGAATTGGTCGACGACATTGATTATTGACTAGTTATTA |
| 3 | bGH_HomR | ccgagctcatcgctaTTAATTAACCATAGAGCCCACCGCATCCCCAGCA |
| 4 | pBAC_HomF | TCAATAATCAATGTcgtcgaccaattctcatgtttg |
| 5 | pBAC_HomR | CGGTGGGCTCTATGGTTAATTAAtagcgatgagcteggacttccattg |
| 6 | 37BAC_loxP_F | agctcatcgctaTTAataacttcgtatagcatacattatacgaagttatTATAACACCCCGCGAA GACG |
| 7 | 37BAC_PacI_R | CCACGAGGTCATGCGGTTTTTAATTAAGATCTCCCGCACGGTGTGCT |
| 8 | 38BAC_PacI_F | GTGCGGGAGATCTTAATTAAAAACCGCATGACCTCGTGGG |
| 9 | 38BAC_loxp_R | GCGGTGGGCTCTATGGTTAataacttcgtataatgtatgctatacgaagttatATTGTCCG GATGTGCGGGCA |
| 10 | pBAC-loxP-VF2 | TAACCATAGAGCCCACCGCATCCCCAGCA |
| 11 | pBAC-loxp-VR2 | TAAtagcgatgagctcggacttccattg |
| 12 | PX330-sgRNA | ttcgcggacccaggcccgg |
| 13 | UL37_iF3 | AAGACTGCCAGTGCCGACGAACTCA |
| 14 | sopC-iR | ACCACGGTCCCACTTGTATTGTCGAT |
| 15 | eGFP_BACinf_F | cttgGTACCGAGCTCGGATCCGCCACCATGGTGAGCAAGGG |
| 16 | UL38-iR3 | GCGGTAAGTGAATACCAGGTACAG |
| 17 | sopB-iF2 | TTCCAGCAAATTCATTCTGCAATCGG |
| 18 | repE-iR1 | GCGATATCACTTCCATGACGACAGG |
| 19 | BAC_seque-iF | AATCCGCTCCACTTCAACGTAACACC |
| 20 | ChlR+_iR3 | CCAGCTCACCGTCTTTCATTGCCAT |

## Claims

1. An isolated HSV-1 clinical viral evolution strain, wherein the strain is designated as C1-31110 and deposited at the China Center for Type Culture Collection (CCTCC) under accession no. CCTCC NO: V202335.

2. The viral evolution strain according to claim 1, wherein the viral evolution strain is obtained by directed evolution of HSV-1 clinical virus strains; and/or
The clinical virus strain is isolated from exudate of blistered tissue of the lips and face of a patient with herpes labialis; and/or
The clinical virus strain is designated as C1-0803-1-1-1 and deposited at CCTCC under accession no. CCTCC NO: V202351; and/or
The viral evolution strain is isolated, purified, and amplified.

3. The viral evolution strain according to claim 1, wherein obtaining said virus evolution strain comprises subjecting the clinical virus strain to successive passages (e.g., 3-10 passages, such as 5 passages); screening the successively passaged virus strain based on plaque phenotype after infecting Vero cells; and isolating a virus strain that produces single, larger viral plaquesthan other virus strains; optionally, purifying the isolated virus strain.

4. An isolated HSV-1 clinical virus strain, wherein the strain is designated as C1-0803-1-1-1, and deposited at CCTCC under accession no. CCTCC NO: V202351.

5. An infectious clone, comprising a viral genome derived from the clinical viral evolution strain according to any one of claims 1-3 or the clinical virus strain according to claim 4, and a bacterial artificial chromosome (BAC) cloning vector backbone sequence inserted into the viral genome.

6. The infectious clone according to claim 5, comprising a viral genomic DNA into which the BAC cloning vector backbone sequence (e.g., pBeloBAC11-CMV-eGFP backbone sequence) is inserted (e.g., the insertion site is viral genome UL37/UL38); and/or
A plasmid map of the infectious clone is shown in Figure 6; and/or
The infectious clone is designated as CB720, and deposited at CCTCC under accession no. CCTCC NO: V202352.

7. A derived virus strain derived from the clinical viral evolution strain of any one of claims 1-3, the clinical virus strain of claim 4, or the infectious clone of claim 5 or 6, wherein the derived virus strain is obtained by subjecting the clinical virus strain, clinical viral evolution strain, or infectious clone to one or more genetic modifications selected from the group consisting of (1) reverse genetic modification (e.g., modification to a viral genome of the infectious clone); (2) homologous recombination; and (3) Cre/LoxP site-specific recombination system modification.

8. The derived virus strain of claim 7, wherein the genetic modification comprises one or more selected from the group consisting of:
Deleting non-essential genes, such as deleting ICP34.5, ICP6, ICP47, functional glycoprotein H encoding gene, functional thymkinase encoding gene; and/or
Modifying promoters of essential genes by replacing them with tumor-specific promoters, enabling the virus to replicate exclusively in cancer cells; and/or
Engineering structural proteins of virus, for example, gB, gD, gH, *etc*., by inserting domains targeting tumor-associated antigens (TAAs), (e.g., scFvs, VHH, or other domains with specific binding capabilities), enabling the virus to specifically bind and infect TAA-expressing cancer cells; and/or
Introducing heterologous genes that improve immune response and/or tumor suppression, such as genes encoding immunostimulatory polypeptides (e.g., GM-CSF, cytokines or chemokines (CCL5, CCL20, CCL21), RNATES, B7.1, B7.2, IL-12, IL-15, HSP70), genes encoding prodrug-activating proteins (e.g., nitroreductase, cytochrome p450), genes encoding tumor suppressor peptides or tumor suppressor proteins (e.g., p53, TRAIL, anti-PD-1 antibody, endostatin), bispecific antibodies, or immune-killing cell engagers (e.g., T-cell engagers, NK -cell engagers); and/or
Introducing heterologous genes that improve viral replication and spread within tumors or modify the tumor microenvironment, such as ECM degradation genes (hyaluronidase).

9. A product comprising the clinical viral evolution strain of any one of claims 1 to 3, the clinical virus strain of claim 4, the infectious clone of claim 5 or 6, and/or the derived virus strain of claim 7 or 8.

10. The product of claim 9, wherein the product is for use as a drug or viral vector vaccine for tumor prevention and/or treatment, a material for pharmaceutical or vaccine production and/or research and development, and/or an exogenous gene vector,
For example, the tumor is selected from solid tumors, such as bladder cancer, liver cancer, lung cancer, breast cancer, melanoma, ovarian cancer, prostate cancer, kidney cancer, intestinal cancer, head and neck cancer, skin cancer, pancreatic cancer, colorectal cancer, squamous cell carcinoma, mesothelioma, myeloma, osteosarcoma, thyroid cancer, cervical cancer, and bile duct cancer; non-solid tumors, such as hematologic malignancies (e.g., leukemia) and lymphoma; and nervous system tumors (e.g., glioblastoma and neuroblastoma); preferably, the tumor is selected from breast cancer, liver cancer, pharyngeal cancer, lung cancer, and malignant glioma.

11. Use of the clinical viral evolution strain of any one of claims 1 to 3, the clinical virus strain of claim 4, the infectious clone of claim 5 or 6, and/or the derived virus strain of claim 7 or 8 in the preparation of a drug or viral vector vaccine for tumor prevention and/or treatment, or a material for drug or vaccine production and/or research and development, and/or an exogenous gene vector,
For example, the tumor is selected from solid tumors, such as bladder cancer, liver cancer, lung cancer, breast cancer, melanoma, ovarian cancer, prostate cancer, kidney cancer, intestinal cancer, head and neck cancer, skin cancer, pancreatic cancer, colorectal cancer, squamous cell carcinoma, mesothelioma, myeloma, osteosarcoma, thyroid cancer, cervical cancer, and bile duct cancer; non-solid tumors, such as hematologic malignancies (e.g., leukemia) and lymphoma; and nervous system tumors (e.g., glioblastoma and neuroblastoma); preferably, the tumor is selected from breast cancer, liver cancer, pharyngeal cancer, lung cancer, and malignant glioma.
